# EUROPEAN PATENT APPLICATION

(11) **EP 2 548 565 A2**
(43) Date of publication of application: **23.01.2013**
(21) Application number: 11744932.2
(22) Date of filing: 21.02.2011
(51) Int. Cl.: A61K 36/39, A61P 3/10, A61P 3/00

(54) **PHARMACEUTICAL COMPOSITION FOR TREATING DIABETES CONTAINING QUAMOCLIT ANGULATA EXTRACTS**

(30) Priority: 19.02.2010 KR 20100014977
(71) Applicant: Korea Research Institute of Bioscience and Biotechnology, Daejeon 305-333 (KR)
(72) Inventor: CHUNG, Bong Hyun, Daejeon 302-280 (KR); KIM, Moon Il, Daejeon 305-806 (KR); YI, So Yeon, Hongsung-gun Chungcheongnam-do 350-900 (KR); LEE, Ui Jin, Daejeon 302-160 (KR); PARK, Kyoungsook, Geumsan-gun Chungcheongnam-do 312-911 (KR); BAE, Pan Kee, Daejeon 305-804 (KR)
(74) Representative: Hiebl, Inge Elisabeth
(86) International application number: PCT/KR2011/001128
(87) International publication number: WO 2011/102690

(57) **Abstract**

The present invention relates to a pharmaceutical composition for treating diabetes and its complications, containing a *Quamoclit angulata* extract, and more particularly to a *Quamoclit angulata* extract, a pharmaceutical composition for preventing or treating diabetes and its complications, a pharmaceutical composition for preventing or delaying aging, and a pharmaceutical composition for preventing or treating cancer, which contain the *Quamoclit angulata* extract. The compositions of the invention exhibit excellent effects on blood glucose lowering, promotion of insulin secretion, reduction of proteinuria, and reduction of lenticular opacity. Thus, the pharmaceutical compositions have excellent effects on the prevention or treatment of diabetes and its complications.

## Description

### TECHNICAL FIELD

The present invention relates to a pharmaceutical composition for treating diabetes and its complications, containing a *Quamoclit angulata* extract, and more particularly to a *Quamoclit angulata* extract, a pharmaceutical composition for preventing or treating diabetes and its complications, a pharmaceutical composition for preventing or delaying aging, and a pharmaceutical composition for preventing or treating cancer, which contain the *Quamoclit angulata* extract.

### BACKGROUND ART

Diabetes refers to a group of metabolic disorders characterized by chronic hyperglycemia resulting from defects in insulin secretion or action. When abnormally high blood glucose levels are continued for a long period time, various complications occur due to chronic metabolic disorders and the resulting chronic vascular injuries.

Diabetes, a typical adult metabolic disease, is suffered by about 5% of the population in the world and causes a huge loss of lives and properties. Most diabetic patients take oral therapeutic agents, but a safe therapeutic agent has not yet been developed. Insulin resistance is known to be the most important cause of diabetes, but the exact mechanism of diabetes is still unknown, and it is known that diabetes is caused by genetic predisposition and environmental factors.

Diabetes is the third leading cause of death in the world, and the number of diabetic patients in 2010 is estimated to be about 250 millions. In Korea, the number of diabetic patients is expected to increase continuously in the future. Non-insulin dependent diabetes (NIDDM) is the seventh leading cause of death in Korea and accounts for more than 90% of diabetic patients. It is called "adult diabetes", because it occurs mainly in persons who are over 40 years old. It is a metabolic disorder which is caused by the insufficient production or inappropriate use of insulin. Although the cause of onset of NIDDM is not yet clearly known, it is believes that NIDDM is caused by environmental factors, including westernized eating habits and life styles, as well as genetic factors such as obesity and a lack of exercise. For treatment of NIDDM, dietary therapy and exercise therapy are first attempted, and if the therapeutic effects of such therapies are insufficient, drugs are used, and in many cases, insulin is used.

Insulin is required for patients whose blood glucose levels are not regulated by dietary therapy and oral blood glucose lowering drugs. However, because insulin is a protein, it is inactivated by hydrolysis in the stomach. For this reason, it cannot be administered orally and should be injected intravenously or subcutaneously.

Oral blood glucose lowering drugs improve the sensitivity of insulin receptor of a cell and stimulate the pancreases to promote secretion of insulin, and thus they are used to regulate blood glucose levels in NIDDM patients.

However, oral therapy for treatment of NIDDM can cause hypoglycemia, nausea, vomiting, diarrhea, eruption and the like. Particularly, it can cause serious adverse effects such as fatal lactic acidosis. In addition, oral blood glucose lowering agents, when used for a long period of time, cause cardiovascular disorders or gastrointestinal and hepatic disorders. For this reason, the long-term use thereof is not recommended.

Due to such shortcomings and adverse effects, among current therapeutic drugs, there are little or no drugs, which show satisfactory effects, have high safety without adverse effects and can be applied to all diabetic patients. Thus, there is an urgent need to develop a more efficient drug for treating diabetes, particularly NIDDM.

About 10 years after the onset of diabetes, almost all the organs of the body are damaged, causing complications. Such complications include acute diseases, such as hypoglycemia, ketoacidosis, hyperosmolar nonketotic hyperglycemia, hyperglycemic coma, and diabetic ketoacidosis; and chronic diseases, such as diabetic retinopathy, diabetic cataract, diabetic nephropathy, diabetic neuropathy, cardiovascular complications, and viral infections. Chronic diabetic nephropathy is the most important cause of hemodialysis and end stage renal failure, and diabetic cataract causes blindness, and eventually leads to death. The mechanisms causing diabetes are generally described by nonenzymatic glycation of proteins, polyol pathways, and the like.

The non-enzymatic glycation of protein is caused by condensation of amino acid group such as lysine residue of protein with reduced sugar without enzymatic action, that is, the Maillard reaction. As a result of the reaction, glycation end product are produced. The non-enzymatic glycation of protein includes two steps. In the first step, an amino acid group (such as lysine of protein) and aldehyde or ketone of reduced sugar are subjected to a nucleophilic addition reaction without enzymatic action to form a Schiff base, a product of the early stage, and the Schiff base is condensed with the adjacent ketoamine adduct to produce a reversible Amadori-type early glycation product. In the second step, as the high blood glucose level is kept, the reversible Amadori type early glycation product is rearranged without degradation and is cross-linked with a protein to form irreversible advanced glycation end products.

Unlike the reversible Amadori type early glycation product, the advanced glycation end products are irreversible products. Therefore, the advanced glycation end products are not degraded, even when the blood glucose level is returned to the normal level, but they are accumulated in tissue to abnormally change the structure and function of the tissue for the survival period of the protein, thus causing complications in the tissue.

For example, glycated albumin which is one of the advanced glycation end products produced by the reaction of glucose with various proteins acts as the major cause of chronic diabetic nephropathy. The glycated albumin is more easily introduced into glomerular cells compared to normal albumin, and a high concentration of glucose stimulates mesangial cells to increase the synthesis of extracellular matrix. The excessively introduced glycated albumin and the increased extracellular matrix cause the fibrosis of glomeruli. By these mechanisms, the glomeruli are continuously damaged, so that extreme treatment methods such as hemodialysis and organ transplantation are necessarily required. In addition, it was reported that, in the case of chronic diabetes, the collagen in arterial walls and the basement membrane protein in glomeruli bind to the advanced glycation end products and are accumulated in tissue.

Due to the non-enzymatic protein glycation as described above, proteins such as the basement membrane, plasma albumin, the crystalline lens protein, fibrin, collagen and the like are glycated. The advanced glycation end products abnormally change the structure and function of the tissue to cause chronic diabetic complications such as diabetic retinopathy, diabetic cataract, diabetic nephropathy, diabetic neuropathy and the like. Thus, it was found that inhibiting the formation of advanced glycation end products is very important in delaying the onset of diabetic complications or preventing or treating diabetic complications.

In addition, advanced glycation end products overexpress vascular endothelial growth factor (VEGF) mRNA and protein to cause non-proproliferative or proliferative diabetic retinopathy. Aberrant angiogenesis or the pathogenic growth of new blood vessels is involved in a number of conditions. Such conditions include diabetic retinopathy, psoriasis, exudative or wet age-related macular degeneration (ARMD), rheumatoid arthritis and other inflammatory diseases, and most cancers. The VEGF in tumors or tissues suffering from diseases associated with these conditions expresses at an aberrantly high level, and has increased angiogenesis or vascular permeability. ARMD in particular is a clinically important angiogenic disease. This condition is characterized by choroidal neovascularization in one or both eyes in aging individuals, and is the major cause of blindness in industrialized countries. Anti-angiogenic agents used in various therapies can produce only a stoichiometric reduction in VEGF or VEGF receptor, and the agents are typically overwhelmed by the abnormally high production of VEGF by the diseased tissue.

Accordingly, the present inventors have made extensive efforts to find a natural herbal substance for treating diabetes and its complications, which has less adverse effects. As a result, the present inventors have found that an extract of *Quamoclit angulata* functions to inhibit VEGF in human retinal epithelial cells and promotes insulin secretion in pancreatic beta-cells, and that when the *Quamoclit angulata* extract is administered to high-fat diet mice and diabetic model mice, the diabetic characteristics of the mice are reduced, thereby completing the present invention.

### DISCLOSURE OF INVENTION

### TECHNICAL PROBLEM

It is an object of the present invention to provide a *Quamoclit angulata* extract which is a natural herbal substance for treating diabetes and its complications.

Another object of the present invention is to provide a pharmaceutical composition for preventing or treating diabetes and its complications, which contains the *Quamoclit angulata* extract.

Still another object of the present invention is to provide a pharmaceutical composition for preventing or delaying aging, which contains the *Quamoclit angulata* extract.

Yet another object of the present invention is to provide a pharmaceutical composition for preventing or treating cancer, which contains the *Quamoclit angulata* extract.

A further object of the present invention is to provide a method for preparing the *Quamoclit angulata* extract.

### TECHNICAL SOLUTION

To achieve the above objects, the present invention provides a pharmaceutical composition for preventing or treating diabetes and its complications, which contains a *Quamoclit angulata* extract.

The present invention also provides a health functional food for preventing diabetes and its complications, which contains a *Quamoclit angulata* extract. The present invention also provides a pharmaceutical composition for preventing or delaying aging, which contains a *Quamoclit angulata* extract.

The present invention also provides a health functional food for preventing or delaying aging, which contains a *Quamoclit angulata* extract.

The present invention also provides a pharmaceutical composition for preventing or treating cancer, which contains a *Quamoclit angulata* extract.

The present invention also provides a method for preparing a *Quamoclit angulata* extract, the method comprising the steps of: (a) finely cutting a whole plant of *Quamoclit angulata* and extracting the cut plant with a solvent selected from the group consisting of water, alcohol, an organic solvent, and mixtures thereof, thereby obtaining an extract; and (b) filtering the extract and concentrating the filtered extract under reduced pressure, thereby obtaining a *Quamoclit angulata* extract.

The method of the present invention may further comprise step (c) of purifying the *Quamoclit angulata* extract, obtained in step (b), using activated carbon, and concentrating the purified extract under reduced pressure.

The present invention provides a *Quamoclit angulata* extract, which is prepared by the above method and has VEGF inhibitory effects, insulin secretion promoting effects, blood glucose lowering effects, proteinuria (urinary protein) lowering effects, and lenticular opacity reducing effects.

The present invention also provides a method for preventing or treating diabetes and its complications, the method comprising administering the *Quamoclit angulata* extract to a subject.

The present invention also provides a method for preventing or delaying aging, the method comprising administering the *Quamoclit angulata* extract to a subject.

The present invention also provides a method for preventing or treating cancer, the method comprising administering the *Quamoclit angulata* extract to a subject.

The present invention also provides the use of the *Quamoclit angulata* extract for treating or preventing diabetes and its complications.

The present invention also provides the use of the *Quamoclit angulata* extract for preventing or delaying aging.

The present invention also provides the use of the *Quamoclit angulata* extract for preventing or treating cancer.

Other features and embodiments of the present invention will be more apparent from the following detailed descriptions and the appended claims

### BRIEF DESCRIPTION OF THE DRAWINGS

FIG. 1 shows the results obtained by examining the effect of a *Quamoclit angulata* extract of the present invention on the inhibition of expression of VEGF (vascular endothelial growth factor) in human retinal epithelial cells.
FIG. 2 shows the inhibition of VEGF protein production in human retinal epithelial cells by a *Quamoclit angulata* extract of the present invention.
FIG. 3 is a graphic diagram showing the stimulation of insulin protein production in mouse pancreatic beta-cells by a *Quamoclit angulata* extract of the present invention.
FIG. 4 shows the effect of a *Quamoclit angulata* extract of the present invention on the weight of high-fat diet mice.
FIG. 5 shows the effect of a *Quamoclit angulata* extract of the present invention on the lowering of blood glucose levels in high-fat diet animals.
FIG. 6 shows the effect of a *Quamoclit angulata* extract of the present invention on the reduction of proteinuria in high-fat diet animals.
FIG. 7 shows the effect of a *Quamoclit angulata* extract of the present invention on the lowering of blood glucose levels in diabetic model animals.
FIG. 8 shows the effect of a *Quamoclit angulata* extract of the present invention on the inhibition of glycated hemoglobin in diabetic model animals.
FIG. 9 shows the effect of a *Quamoclit angulata* extract of the present invention on the reduction of proteinuria in diabetic model animals.
FIG. 10 shows the effect of a *Quamoclit angulata* extract of the present invention on the reduction in the opacity of mouse lenses.

### BEST MODE FOR CARRYING OUT THE INVENTION

Unless defined otherwise, all technical and scientific terms used herein have the same meaning as commonly understood by one of ordinary skill in the art to which the invention pertains. Generally, the nomenclature used herein and the experiment methods which will be described later are those well known and commonly employed in the art.

Hereinafter, the present invention will be described in detail.

In one aspect, the present invention is directed to a pharmaceutical composition and a health functional food for treating or treating diabetes and its complications, which contains a *Quamoclit angulata* extract.

*Quamoclit angulata* that is used in the present invention is a annual vine plant belonging to the family *Convolvulaceae* of the order *Tubiflorae.* It is native to tropical Africa and is cultivated mainly for ornamental purposes. It is characterized in that the vines grow like a morning glory and are rolled up on the left side. The whole plant has a length of about 3 m. The leaves are alternate with long petioles and have a heart shape. The leaf tips are acute, and both ends of the lower portion of the leaf are pointed. The flowers bloom between August and September and are yellowish red, and 3-5 flowers are suspended from each stalk. The flower looks similar to a morning glory and has 5 sepals, 5 stamens and 1 pistil. The fruit is capsular, ripens in September and has a sepal remaining thereon. This plant is similar to *Quamoclit coccinea*, but the leaf is not divided.

A *Quamoclit angulata* extract according to the present invention can be prepared using a known method. For example, *Quamoclit angulata* can be extracted with a solvent selected from among water, alcohol, an organic solvent, and mixtures thereof. Preferably, the plant may be extracted using water or alcohol. In addition, the extract can be purified using activated carbon.

In one aspect of the present invention, the *Quamoclit angulata* extract was obtained in the following manner. 51 g of crushed *Quamoclit angulata* was extracted with a solvent selected from the group consisting of water, alcohol, an aqueous alcohol solution, an organic solvent, and mixtures thereof, in an ultrasonic extractor at room temperature for 15 minutes at 2-hr intervals for 2-3 days. The resulting extract was extracted under reduced pressure in a rotary vacuum evaporator at room temperature, and the concentrator was dried in a vacuum freeze dryer and then dissolved in water, thereby obtaining a *Quamoclit angulata* extract.

In addition, the obtained *Quamoclit angulata* extract was passed through an activated carbon-packed column to adsorb the active components thereof onto the activated carbon. Then, the activated carbon-packed column was washed with distilled water to remove non-adsorbed components. Then, to the activated carbon-packed column from which the non-adsorbed components have been removed, an organic solvent such as 10-50% (v/v) ethanol was added while increasing the concentration continuously or stepwise, so that the active components of *Quamoclit angulata* adsorbed onto the activated carbon were purified by elution. Then, the *Quamoclit angulata* extract was collected. The *Quamoclit angulata* extract purified as described above was concentrated under reduced pressure in a rotary vacuum evaporator at room temperature, and the concentrated extract was freeze-dried in a vacuum, and then dissolved in water, thereby obtaining an aqueous solution of a *Quamoclit angulata* extract.

In one Example of the present invention, weight, blood glucose and proteinuria, which are diabetic characteristics, were examined. The *Quamoclit angulata* extract was administered to high-fat diet-induced obesity mice for 4 weeks, and as a result, it could seen that the extract exhibited blood glucose lowering activity and reduced urinary protein (an early sign of diabetic nephropathy), suggesting that the *Quamoclit angulata* extract according to the present invention can prevent diabetes.

In another Example of the present invention, the *Quamoclit angulata* extract was administered to diabetic mice (male C57BL/Ks DB/DB mice) for 12 weeks. As a result, it could be seen that the extract exhibited strong blood glucose lowering activity and lowered the level of glycated hemoglobin. In addition, the *Quamoclit angulata* extract of the present invention reduced proteinuria. This suggests that the *Quamoclit angulata* extract of the present invention can treat diabetes.

Moreover, whether the *Quamoclit angulata* extract of the present invention has hepatotoxicity in mice was examined, and as a result, it was found that showed no hepatotoxicity. This suggests that, when the extract of the present invention is administered to mammals, it can significantly ameliorate, prevent and treat diabetes and its complications.

As used herein, the term "preventing" or "treating" is intended to include preventing or treating diabetes or its complications, or ameliorating or improving the conditions caused by diabetes or its complications.

In addition, the *Quamoclit angulata* extract of the present invention can prevent or treat not only diabetes, but also diabetic complications which are selected from the group consisting of chronic hyperglycemia, atherosclerosis, microangiopathy, renal diseases, cardiac diseases, diabetic retinopathy, and other ocular diseases. In another Example of the present invention, whether the *Quamoclit angulata* extract of the present invention inhibits the development of a cataract (diabetic complication) was examined by culturing mouse lenses. As a result, it was found that the *Quamoclit angulata* extract of the present invention inhibited the development of a cataract.

As used herein, the term "diabetes" is intended to include all types of diabetes, including type 1 diabetes, type 2 diabetes, adult diabetes occurring in young persons, latent autoimmune diabetes, and pancreatic diabetes, as well as diabetic complications, including hyperglycemia, hyperinsulinemia, impaired glucose tolerance, impaired fasting glucose, dyslipidemia, hypertriglyceridemia or insulin resistance.

As used herein, the term "ocular diseases" is intended to include, in addition to diabetic retinopathy, all ocular diseases caused by diabetes, including a cataract, macular degeneration, external ophthalmoplegia, iridocyclitis, neuritis, glaucoma, retinal degeneration, fundus hemorrhage, ametropia, subconjunctival hemorrhage, and vitreous haemorrhage.

The pharmaceutical composition of the present invention contains a *Quamoclit angulata* extract as active ingredient, and may be provided as a pharmaceutical composition containing a *Quamoclit angulata* extract alone or in combination with at least one pharmaceutically acceptable carrier, excipient or diluent. The pharmaceutical composition of the present invention may also be used in combination with an agent for treating diabetes or its complications, known in the art. The dosage of the *Quamoclit angulata* extract of the present invention may vary depending on the patient's age, sex and weight, but it may be administered at a dosage of 0.01-200 mg/kg, preferably 0.1-40 mg/kg, once or several times a day. In addition, the *Quamoclit angulata* extract may be contained in the pharmaceutical composition in a pharmaceutically effective amount depending on the kind of diabetes and its complications and its severity, the patient's age, weight, health condition and sex, the route of administration and the period of treatment.

As used herein, the term "pharmaceutically acceptable composition" refers to a composition that is physiologically acceptable and does not cause gastric disorder, allergic reactions such as gastrointestinal disorder or vertigo, or similar reactions, when administered to humans. Examples of the pharmaceutically acceptable carriers may include carriers for oral administration such as lactose, starch, cellulose derivative, magnesium stearate, stearic acid, and the like, and carriers for parenteral administration such as water, suitable oil, saline solution, aqueous glucose, glycol, and the like. The pharmaceutical composition of the present invention may additionally contain stabilizers, fillers, anti-aggregating agents, lubricants, wetting agents, perfumes, emulsifiers and preservatives. Examples of suitable stabilizers include sodium hydrogen sulfite, sodium sulfite, or antioxidants such as ascorbic acid. Examples of suitable preservatives include benzalkonium chloride, methyl- or prophyl-paraben, and chlorobutanol. The following document may be referred to for other examples of other the pharmaceutically acceptable carriers: (Remington's Pharmaceutical Sciences, 19th ed., Mack Publishing Company, Easton, PA, 1995).

In addition, the pharmaceutical composition of the present invention may be formulated using a method well known in the art, such that it can provide the rapid, sustained or delayed release of the active ingredient after administration to mammals. The pharmaceutical composition of the present invention may be in the form of powders, granules, tablets, emulsions, syrups, aerosols, soft or hard gelatin capsules, sterile injection solutions, sterile powders, etc.

The pharmaceutical composition of the present invention may be administered by various routes to mammals, including rats, mice, livestock and humans. All routes of administration can be contemplated and include, for example, oral, tissue, rectal, intravenous, intramuscular, intrauterine, intrathecal or intracerebrovascular injections. Further, the pharmaceutical composition of the present invention may be administered in combination with a known compound having diabetes prevention and treatment effects

Thus, the present invention is directed to a method for preventing or treating diabetes and its complications, the method comprising administering a pharmaceutical composition containing the *Quamoclit angulata* extract as an active ingredient to a mammal at a therapeutically effective amount.

In addition, the *Quamoclit angulata* extract according to the present invention may be prepared in the form of foods for the purpose of preventing or improving diabetes and its complications. Thus, the present invention is directed to a health functional food as a food composition containing the *Quamoclit angulata* extract as an active ingredient.

The health functional food of the present invention is a food composition, and examples thereof include all types of food including functional food, nutritional supplement, health food, and food additives. The above types of food may be prepared in various forms by conventional methods known in the art. For example, for the health food, the *Quamoclit angulata* extract of the present invention may be prepared in the form of tea, juice and drink, or it may be granulized, encapsulated or powdered. Also, the functional food may be prepared by adding the *Quamoclit angulata* extract of the present invention to beverages (including alcoholic beverage), fruits and processed foods thereof (e.g., canned fruit, bottled food, jam, marmalade, etc.), fishes, meats and processed foods thereof (e.g., ham, sausage, corn beef, etc.), bread and noodles (e.g., Japanese noodle, buckwheat noodles, ramen, spaghetti, macaroni, etc.), fruit juice, various drinks, cookies, taffy, milk products (e.g., butter, cheese, etc.), edible plant oil and fat, margarine, vegetable proteins, a retort food, frozen food, various seasonings (e.g., soybean paste, soy sauce, sauces, etc.) and the like.

In another aspect, the present invention is directed to a pharmaceutical composition and a health functional food for preventing or delaying aging, which contains a *Quamoclit angulata* extract.

Studies related diabetes and diabetic complications reported that diabetes has a close connection with oxidative stress. Chronic hyperglycemia appearing in diabetes increases the production of free radicals by various pathways, including glucose autoxidation and protein glycation, and these highly reactive substances increase oxidative stress. The *Quamoclit angulata* extract of the present invention can inhibit the production of advanced glycation end products (AGEs) and reduce the induction of oxidative stress caused by these substances, thereby exhibiting the effects of preventing and delaying aging.

The pharmaceutical composition of the present invention may include pharmaceutically acceptable carriers or excipients and may be used in combination with an agent for delaying or preventing aging, known in the art. In addition, the dosage of the *Quamoclit angulata* extract of the present invention may vary depending on the degree of aging, the desired effect, the patient's age, sex and weight, the period of administration, etc. Moreover, the *Quamoclit angulata* extract of the present invention may be formulated in various forms using a known technique and may be administered by various routes. Accordingly, the present invention is directed to a method for preventing or delaying aging, the method comprising a step of the *Quamoclit angulata* extract to a subject. In addition, the the health functional food of the present invention is a food composition, and examples thereof include various types of food such as functional food, nutritional supplement, health food and food additives. For example, the *Quamoclit angulata* extract of the present invention may be prepared in the form of tea, juice and drink, or it may be granulized, encapsulated or pulverized. Also, the *Quamoclit angulata* extract of the present invention may be added to various foods, including beverages, fruits and processed foods thereof, fishes, meats and processed foods thereof, bread, noodles, various seasonings and the like.

In still another aspect, the present invention is directed to a pharmaceutical composition for preventing or treating cancer, which contains a *Quamoclit angulata* extract.

The *Quamoclit angulata* extract of the present invention can inhibit the production of advanced glycation end products and inhibit carcinogenesis caused by these substances, thereby exhibiting the effect of preventing and/or treating cancer. Because advanced glycation end products were reported to induce aging and carcinogenesis (Tokuda, H., et al., Book of Abstract of 53rd GA Congress joint with SIF, P076, 2005), it can be seen that the *Quamoclit angulata* extract can prevent or treat cancer by inhibiting the production of VEGF.

As used herein, the term "cancer" refers to a disease associated with angiogenesis induced by VEGF (Vascular Endothelial Growth Factor), and examples thereof include all kinds of known cancers, including lung cancer, stomach cancer, liver cancer and pancreatic cancer. As used herein, the expression "preventing or treating cancer" means inhibiting VEGF production to prevent the proliferation and metastasis of cancer and to prevent cancer.

This pharmaceutical composition may include pharmaceutically acceptable carriers or excipients and may be used in combination with an agent for preventing or treating cancer, known in the art. In addition, the dosage of the *Quamoclit angulata* extract may vary depending on the kind of cancer, the degree of progression of cancer, the patient's age, sex and weight, and the period of administration. Additionally, the *Quamoclit angulata* extract may be formulated in various forms using a known technique and may be administered by various routes.

Thus, the present invention is directed to a method for preventing or treating cancer, the method comprising administering the *Quamoclit angulata* extract to a subject.

In yet another aspect, the present invention is directed to a method for preparing a *Quamoclit angulata* extract, the method comprising the steps of: (a) finely cutting a whole plant of *Quamoclit angulata* and extracting the cut plant with a solvent selected from the group consisting of water, alcohol, an organic solvent, and mixtures thereof, thereby obtaining an extract; and (b) filtering the extract and concentrating the filtered extract under reduced pressure, thereby obtaining a *Quamoclit angulata* extract.

The organic solvent in step (a) of the method may be any extraction solvent known in the art, such as hexane, ether, benzene, ethyl acetate, chloroform, acetonitrile, or methanol. Water and alcohol were used as the organic solvent in one Example of the present invention, but are not limited thereto.

The method of the present invention may further comprise step (c) of purifying the *Quamoclit angulata* extract, obtained in step (b), using activated carbon, and concentrating the purified extract under reduced pressure.

Step (c) is a step of recovering active components from the *Quamoclit angulata* extract using activated carbon, filtering the recovered components, and concentrating the filtered components under reduced pressure. Specifically, purification with the activated carbon in step (c) may be performed by suspending the concentrated extract, obtained in step (b), in water, and purifying the suspended extract by an activated carbon-packed chromatography column using 50% ethanol as a mobile phase.

In a further aspect, the present invention is directed to a *Quamoclit angulata* extract, which is prepared by the above method and has VEGF inhibitory effects, insulin secretion promoting effects, blood glucose lowering effects, proteinuria (urinary protein) lowering effects, and lenticular opacity reducing effects.

### EXAMPLES

Hereinafter, the present invention will be described in further detail with reference to examples. It will be obvious to a person having ordinary skill in the art that these examples are illustrative purposes only and are not to be construed to limit the scope of the present invention.

### Example 1: Preparation of Quamoclit angulata extract

*Quamoclit angulata* was collected from Seogwang-ri, Andeok-myeon, Nam-gun, Jeju-do, Korea. 51 g of the *Quamoclit angulata* was extracted with 100% alcohol in an ultrasonic extractor at room temperature for 15 minutes at 2-hr intervals over 2-3 days. The resulting extract was concentrated in a rotary vacuum evaporator at room temperature under reduced pressure, and the concentrate was dried in a freeze vacuum dryer. 7 g of the dried *Quamoclit angulata* extract was dissolved in water at a concentration of 0.075 mg/ml.

### Example 2: Preparation of Quamoclit angulata extract using activated carbon

50 g of *Quamoclit angulata,* collected from Seogwang-ri, Andeok-myeon, Nam-gun, Jeju-do, Korea, was finely cut. The finely cut *Quamoclit angulata* was extracted with water as a solvent in an ultrasonic extractor at room temperature for 15 minutes at 2-hr intervals over 2-3 days. The extract was filtered and concentrated under reduced pressure. The resulting concentrate was suspended in water and purified by elution with an activated carbon-packed chromatography column using 50% ethanol as a mobile phase. The purified *Quamoclit angulata* extract was concentrated under reduced pressure, and 5 g of the concentrated extract was dissolved in water at a concentration of 20 mg/ml.

### Test Example 1: Examination of VEGF inhibitory activity of Quamoclit angulata extract

In order to examine the inhibitory activity of the *Quamoclit angulata* extract prepared in Examples 1 and 2 against vascular endothelial growth factor (VEGF) known to cause diabetic complications such as diabetic retinopathy, human retinal epithelial cells (ARPE-19) were treated with each of the extracts.

Specifically, ARPE 19 cells (ATCC, USA) were cultured in DMEM F12 media supplemented with 10% FBS (fetal bovine serum). To induce the expression of VEGF mRNA and protein in the ARPE 19 cells, the cells were seeded onto a 60-well plate at a density of 1x 10⁵ cells/well, and 10% FBS-containing DMEM low glucose medium was added thereto, and the cells were cultured for 24 hours. Then, the medium was replaced with DMEM low glucose medium containing 25 mM or 30 mM glucose, and each of the *Quamoclit angulata* extracts of Example 1 and 2 was added to the culture medium to a final concentration of 0.2 *µ*g/ml. Then, the cells were cultured for 24 hours in order to examine the inhibition of expression of VEGF mRNA and were cultured for 72 hours in order to examine the production of VEGF protein. Meanwhile, for use as a control, cells were treated with 5.5 mM glucose such that VEGF was not expressed. The degrees of expression of VEGF in the groups treated with 25 mM and 30mM glucose were determined relative to the control, thereby comparing the VEGF inhibitory activities of the *Quamoclit angulata* extracts. The 25 mM and 30 mM glucose concentrations are the concentrations at which the expression of VEGF is optimally induced.

### (1) Inhibitory effect on expression of VEGF mRNA

After the medium has been removed from the ARPE 19 cultured as described above, total RNA was isolated from the cells using TRIzol reagent (Invitrogen, USA). The isolated RNA was subjected to RT-PCR (reverse transcriptase-polymerase chain reaction) using primers of SEQ ID NOS: 1 and 2, thereby examining the expression of VEGF mRNA.

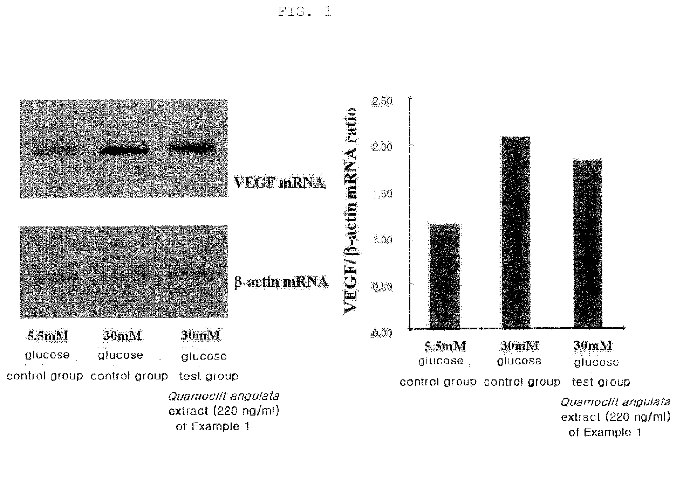

As a result, as can be shown in FIG. 1, the expression of VEGF mRNA in the test group treated with the *Quamoclit* angulata extract of Example 1 was inhibited.
SEQ ID NO: 1: TTGCCTTGCTGCTCTACCTC
SEQ ID NO: 2: AAATGCTTTCTCCGCTCTGA

### (2) Inhibitory effect on expression of VEGF secretory protein

**The** culture medium of the ARPE 19 cells cultured as described above was collected, and the amount of VEGF in the collected culture medium was measured using VEGF ELISA kit (R&D, UK).

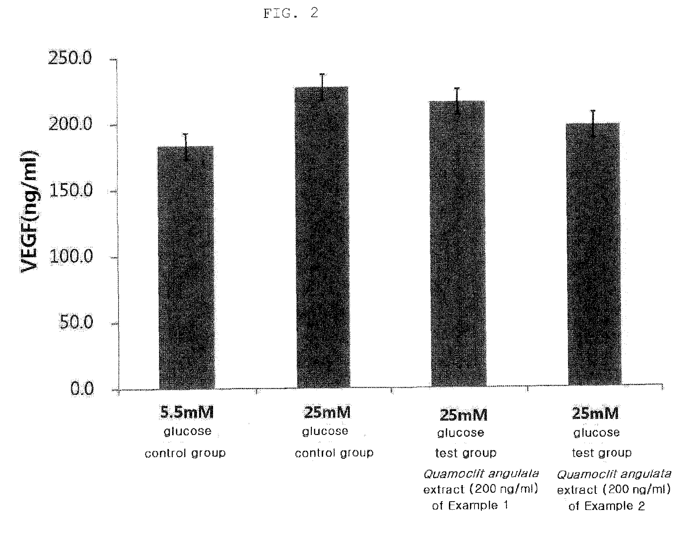

As a result, as can be seen in FIG. 2, the expressions of VEGF in the test groups treated with the *Quamoclit angulata* extracts of Examples 1 and 2 were reduced.

### Test Example 2: Examination of effect of Quamoclit angulata extract on stimulation of insulin secretion

In order to examine whether the *Quamoclit angulata* extracts prepared in Examples 1 and 2 stimulate insulin secretion, mouse pancreatic beta-cells (Min 6) were treated with each of the extracts.

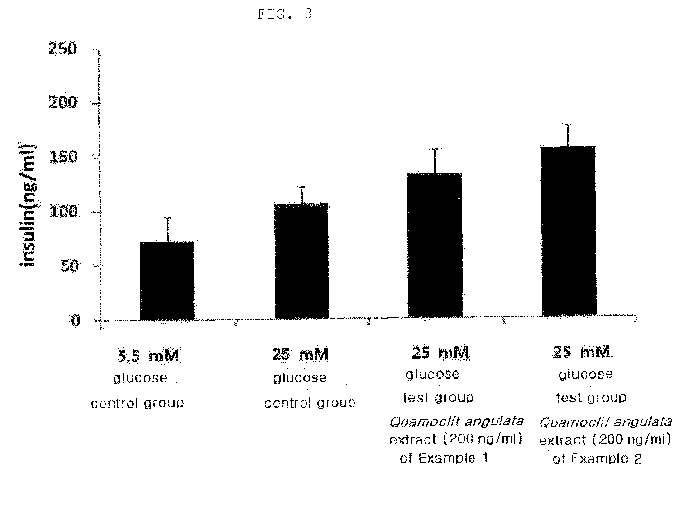

Specifically, Min 6 cells (ATCC, USA) were cultured in HDMEM medium containing 15% FBS (fetal bovine serum). To induce the expression of insulin in the Min 6 cells, the cells were seeded onto a 96-well plate at a density of 1× 10⁴ cells/well, and 15% FBS-containing DMEM high glucose medium was added thereto, and the cells were cultured for 72 hours. Then, the culture plate was washed with PBS buffer and incubated in HBSS buffer for 2 hours. Then, the medium was replaced with 25 mM glucose-containing HBSS buffer, and each of the *Quamoclit angulata* extracts was added to a final concentration of 0.2 *µ*g/ml. Then, the cells were cultured for 2 hours in order to examine insulin expression was stimulated. For use as a control, cells were treated with 5.5 mM glucose such that insulin expression was not induced. The expression of insulin in the group treated with 25 mM glucose was examined, thereby determining whether the *Quamoclit angulata* extract stimulated insulin expression. The culture medium of the Min 6 cells cultured as described above was collected, and the amount of insulin in the collected culture medium was measured using insulin ELISA kit (R&D, UK). As a result, as can be seen in FIG. 3, the expression of insulin in the groups treated with the *Quamoclit angulata* extract was increased. Particularly, the the *Quamoclit angulata* extract of Example 2, prepared using activated carbon, showed a higher insulin secretory activity.

### Test Example 3: Measurement of change in diabetic characteristics of mice by treatment with Quamoclit angulata extract

In order to examine the change in diabetic characteristics, including weight, blood glucose and proteinuria, normal mice were treated with the *Quamoclit angulata* extract prepared in Example 1.

5-week-old mice (male C57BL/6J, 19 g, Koatech, Pyeongtaek, Korea) were purchased and acclimatized under the conditions of constant temperature (25 °C) and humidity (50%) for 1 week before use in the test. The mice were divided into the following groups, each consisting of 4 animals: a control group fed with general feed (general diet group); a high-fat diet group fed with high-fat feed and water; and a test group fed with high-fat diet and an aqueous solution of 0.075 mg/ml of the *Quamoclit angulata* extract of Example 1. The mice of each group were bred for 4 weeks. The changes in the weight and blood glucose of the mice were measured weekly, and 4 weeks after the start of administration, the urine was collected and the urinary protein was measured.

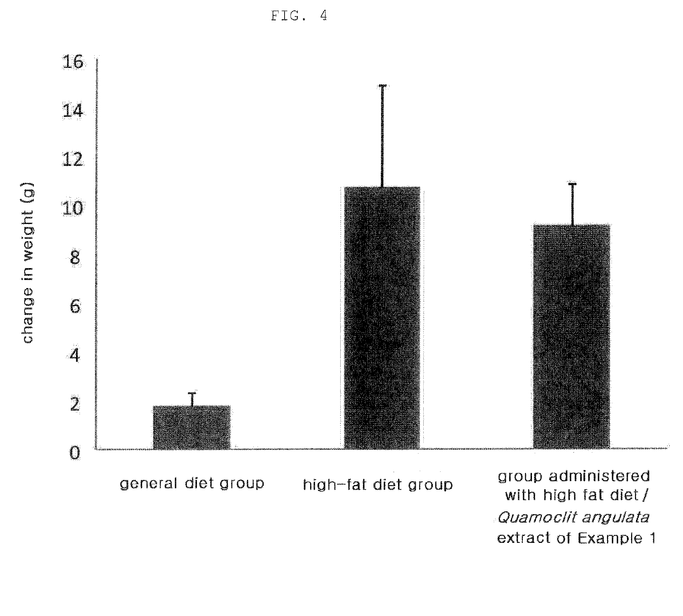

As a result, as can be seen in FIG. 4, the weight of the test group administered with the *Quamoclit angulata* extract significantly decreased compared to the high-fat diet group.

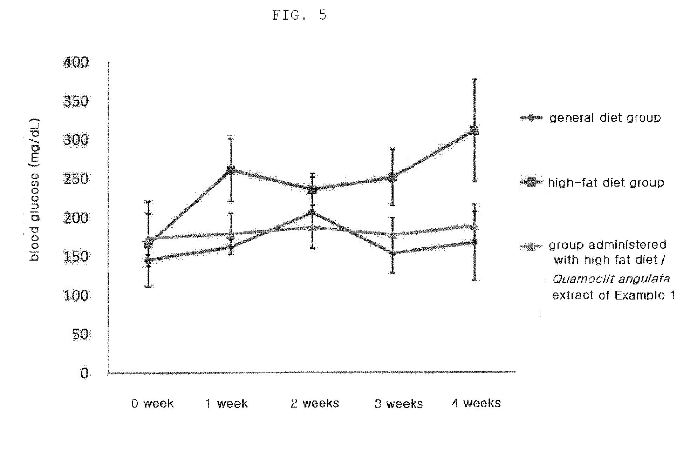

In addition, the blood glucose level was higher in the high-fat diet group than in the general diet group, but was significantly lowered in the group administered with the high-fat diet together with the aqueous solution of 0.075 mg/ml of the *Quamoclit angulata* extract (FIG. 5).

Diabetic patients often indicate renal and cardiac complications. A decrease in proteinuria means the restoration of renal function. In order to examine the effect of the *Quamoclit angulata* extract on the kidneys, urine was sampled from the mice, administered with the *Quamoclit angulata* extract for 4 weeks, and the concentration of protein in the sampled urine was measured using an ELISA kit (Exocell, America).

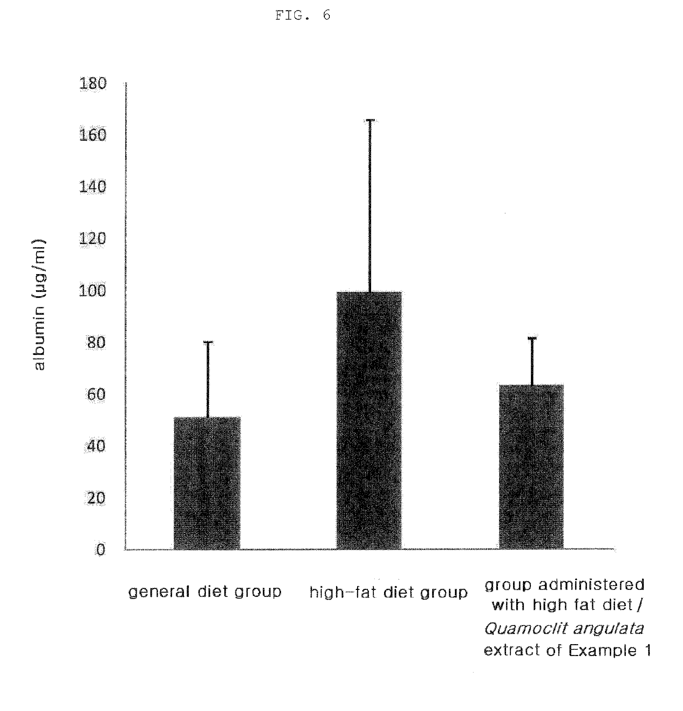

As a result, as can be seen in FIG. 6, the urinary protein concentration was higher in the high-fat diet group than in the general diet group, but was significantly lowered in the group administered with the high-fat diet together with the aqueous solution of 0.075 mg/ml of the *Quamoclit angulata* extract. This suggests that the *Quamoclit angulata* extract improves the renal function characteristic of the mice having high-fat diet-induced diabetes, thereby reducing the urinary protein concentration of the mice.

### Test Example 4: Observation of diabetic characteristics of diabetic mice by treatment with Quamoclit angulata extract

In order to examine the change in diabetic characteristics, including weight, blood glucose and proteinuria, normal mice and diabetic mice were treated with the *Quamoclit angulata* extract prepared in Example 2.

6-week-old mice (male C57BL/6J mice, 20 g, Jungang Lab Animal Inc., Seoul) and 6-week-old diabetic mice (male C57BL/Ks DB/DB mice, 20 g, Jungang Lab Animal Inc., Seoul) were purchased and acclimatized under the conditions of constant temperature (25 °C) and humidity (50%) for 1 week before use in the test. The mice were divided into the following groups, each consisting of 5 animals: a control group consisting of normal mice; a control group consisting of diabetic mice; and a test group consisting of diabetic mice fed with an aqueous solution of 0.1 mg/ml of the *Quamoclit angulata* extract of Example 2. The mice of each group were bred for 12 weeks. The change in the blood glucose level of the mice was measured at 2-week intervals, and the urine was sampled at 6-week intervals and the urinary protein concentration was measured. In addition, after 12 weeks of breeding, blood was collected and the concentration of glycated hemoglobin in the collected blood was measured.

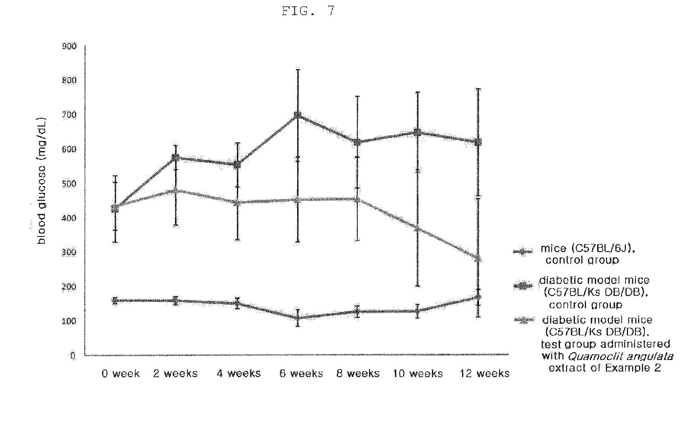

As a result, as can be shown in FIG. 7, the blood glucose level was higher in the diabetic mouse group than in the normal mouse group, but was significantly lowered in the group administered with the aqueous solution of 0.1 mg/ml of the *Quamoclit angulata* extract of Example 2.

In order to reduce the occurrence of complications in diabetic patients, it is important to maintain the blood glucose levels at suitable levels. Blood glucose levels measured at one time point can change due to various factors. For this reason, the pattern of long-term change in blood glucose levels is examined, and for this purpose, the examination of glycated hemoglobin (HbA1c) is most widely used. Glycated hemoglobin consists of glucose bound to hemoglobin normally existing in red blood cells, and as the blood glucose level increases, and the glycated hemoglobin level also increases. Because the glycated hemoglobin level reflects the average blood glucose level for 2-4 months, it is useful to examine the pattern of long-term blood glucose levels.

In order to examine the effect of the *Quamoclit angulata* extract on the glycated hemoglobin level, blood was collected from each of the normal mouse group, the diabetic mouse group and the group administered with the aqueous solution of 0.1 mg/ml of the *Quamoclit angulata* extract of Example 2, once at 6-week intervals, and the level of glycated hemoglobin in the collected blood was measured using an ELISA kit (Cusabio Biotech, Japan).

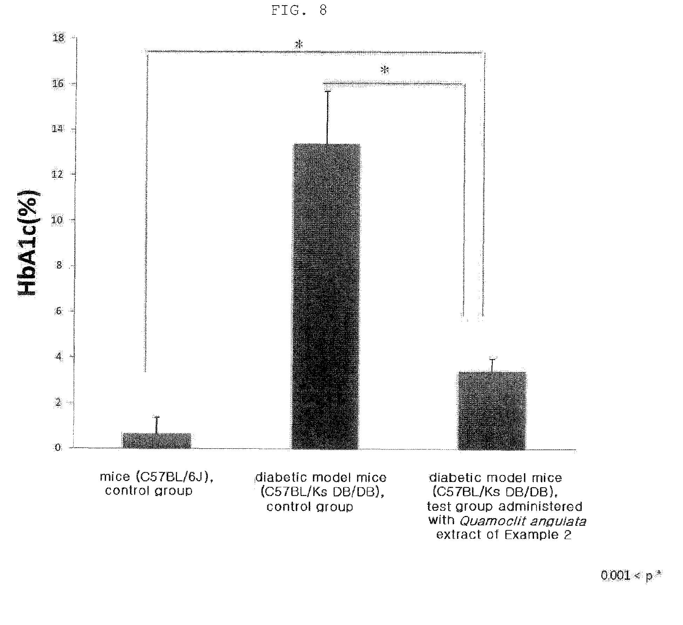

As a result, as can be seen in FIG. 8, the level of glycated hemoglobin was abnormally higher in the diabetic mouse group than in the normal mice group, but was significantly lower in the group administered with the aqueous solution of 0.1 mg/ml of the *Quamoclit angulata* extract of Example 2, than in the diabetic mouse group.

Diabetic patients often indicate renal and cardiac complications. A decrease in proteinuria means the restoration of renal function. In order to examine the effect of the *Quamoclit angulata* extract on the kidneys, urine was sampled from the mice, administered with the *Quamoclit angulata* extract of Example 2 for 12 weeks, and the concentration of protein in the sampled urine was measured using an ELISA kit (Exocell, America).

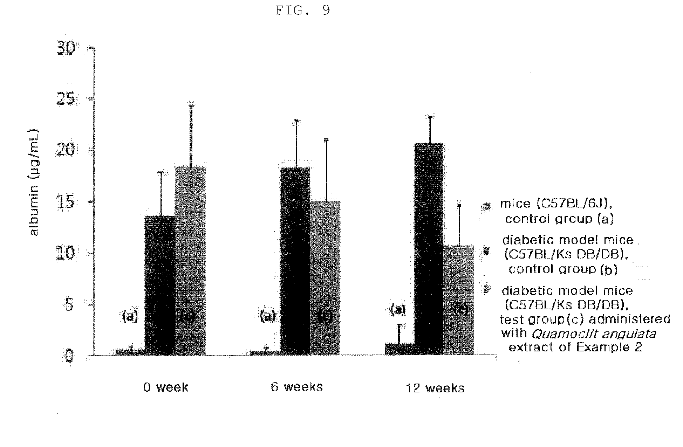

As a result, as can be seen in FIG. 9, the urinary protein level was higher in the diabetic mouse group than in the normal mouse group, but was significantly lower in the group administered with the aqueous solution of 0.1 mg/ml of the *Quamoclit angulata* extract of Example 2, compared than in the diabetic mouse group.

### Test Example 5: Examination of hepatocytotoxicity in diabetic mice

6-week-old mice and 6-week-old diabetic mice were purchased and acclimatized under the conditions of constant (25 °C) and humidity (50 %) for 1 week, and then subjected to a hepatocytotoxicity assay.

The mice were divided into the following groups, each consisting of 5 animals: a control group consisting of normal mice; a control group consisting of diabetic mice; and a test group consisting of diabetic mice fed with an aqueous solution of 0.1 mg/ml of the *Quamoclit angulata* extract of Example 2. The mice of each group were bred for 12 weeks. After 12 weeks of breeding, blood was collected from the mice, and the concentration of alanine transaminase in the collected blood was measured.

Alanine transaminase, an amino-acid metabolic enzyme, is typically used in diagnosis and observation of progression. Maintained concentration of alanine transaminase indicates that the liver was not damaged.

In order to examine the effect of the *Quamoclit angulata* extract on the liver, the mice were bred during 12 weeks, and then blood was collected from each of the normal mouse group, the diabetic mouse group and the group administered with the aqueous solution of 0.1 mg/ml of the *Quamoclit angulata* extract of Example 2, and the concentration of alanine transaminase in the collected blood was measured using an ELISA kit (Cusabio biotech, Japan).

As a result, as can be seen in Table 1 below, the concentration of alanine transaminase was similar between the normal mouse group and the group administered with the aqueous solution of 0.1 mg/ml of the *Quamoclit angulata* extract of Example 2, suggesting that the *Quamoclit angulata* extract has no hepatocytotoxicity.

**[Table 1]**

| Results of measurement of hepatocytotoxicity in diabetic mice after 12 weeks of administration | |
|---|---|
| Group | Concentration of alanine transaminase |
| Mice control group (C57BL/6J) | 35.97±2.73 |
| Diabetic mice control group (C57BL/KsDB/DB) | 37.28±1.89 |
| Diabetic mice test group (C57BL/KsDB/DB) | 35.90±1.32 |
| (Administration of *Quamoclit angulata* prepared in Example 2) | |
| (Unit: IU/L) | |

### Test Example 6: Examination of hepatocytotoxicity in mice

6-week-old mice (Male C57BL/6J mouse, 20 g, Jungang Lab. Animal Inc., Seoul) were purchased and acclimatized under the conditions of constant (25 °C) and humidity (50 %) for 1 week, and then subjected to a hepatocytotoxicity assay.

The mice were divided into the following groups, each consisting of 5 animals: a control group; and a group administered with the *Quamoclit angulata* extract of Example 2. The *Quamoclit angulata* extract was administered to the mice at a dose of 1000 mg/kg three times, after which blood was collected from the mice, and concentration of alanine transaminase in the collected blood was measured.

Alanine transaminase, an amino-acid metabolic enzyme, is typically used in diagnosis and observation of progression. Maintained concentration of alanine transaminase indicates that the liver was not damaged. In order to examine the effect of the *Quamoclit angulata* extract on the liver, blood was collected from the group administered with the *Quamoclit angulata* extract, and the concentration of alanine transaminase in the collected blood was measured using an ELISA kit.

As a result, as can be seen in Table 2 below, the concentration of alanine transaminase was similar between the mouse group and the group administered with the *Quamoclit angulata* extract, suggesting that the *Quamoclit angulata* extract has no hepatocytotoxicity.

**[Table 2]**

| Results of measurement of hepatocytotoxicity in mice | |
|---|---|
| Group | Concentration of alanine transaminase |
| Mice control group (C57BL/6J) | 37.57±1.52 |
| Mice test group (C57BL/KsDB/DB) | 38.18±1.58 |
| Administration of *Quamoclit angulata* prepared in Example 2 at a dose of 1000 mg/kg | |
| (Unit: mIU/ml) | |

### Test Example 7: Incubation of mouse lenses

In the case of diabetic patients, a cataract begins early and grows worse rapidly, resulting in a rapid decrease in the vision. When a person is attacked by diabetes, the lenticular opacity is stimulated. Thus, whether the *Quamoclit angulata* extract of the present invention inhibits the development of a cataract was examined by a lens incubation test.

Eyes were removed from mice and sterilized in an iodine solution for a short time, and lenses were removed from the eyes. The lenses were added to M199 medium and incubated in a cell incubator. During the incubation, 20 mM xylose was added to the medium to induce lenticular opacity, and the lenticular opacity was measured using a CCD camera.

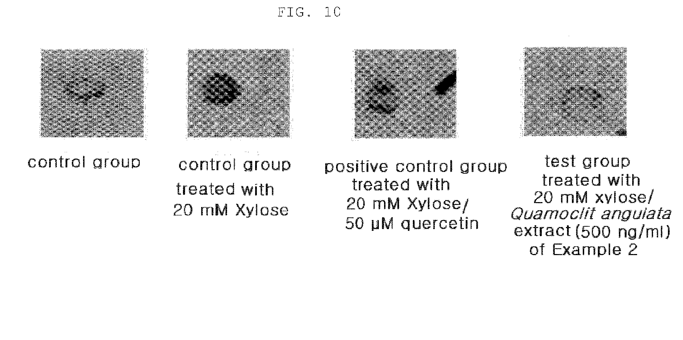

As a result, as can be seen in FIG. 10, the lenticular opacity was lower in the group, administered with the *Quamoclit angulata* extract of Example 2, than a positive control group treated with quercetin known to inhibit the development of a cataract.

### INDUSTRIAL APPLICABILITY

As described above, The compositions of the invention exhibit excellent effects on blood glucose lowering, promotion of insulin secretion, reduction of proteinuria, and reduction of lenticular opacity. Thus, the pharmaceutical compositions have excellent effects on the prevention or treatment of diabetes and its complications.

Although the present invention has been described in detail with reference to the specific features, it will be apparent to those skilled in the art that this description is only for a preferred embodiment and does not limit the scope of the present invention. Thus, the substantial scope of the present invention will be defined by the appended claims and equivalents thereof.

## Claims

1. A pharmaceutical composition for preventing or treating diabetes and its complications, which contains a *Quamoclit angulata* extract.

2. The pharmaceutical composition of claim 1, wherein the extract is extracted with a solvent selected from the group consisting of water, alcohol, an organic solvent, and mixtures thereof.

3. The pharmaceutical composition of claim 1, wherein the diabetic complications are selected from the group consisting of chronic hyperglycemia, atherosclerosis, microangiopathy, renal diseases, cardiac diseases, diabetic retinopathy, and other ocular diseases.

4. A health functional food for preventing diabetes and its complications, which contains a *Quamoclit angulata* extract. (

5. The health functional food of claim 4, wherein the extract is extracted with a solvent selected from the group consisting of water, alcohol, an organic solvent, and mixtures thereof.

6. The health functional food of claim 4, wherein the diabetic complications are selected from the group consisting of chronic hyperglycemia, atherosclerosis, microangiopathy, renal diseases, cardiac diseases, diabetic retinopathy, and other ocular diseases.

7. A method for preparing a *Quamoclit angulata* extract, the method comprising the steps of:
(a) finely cutting a whole plant of *Quamoclit angulata* and extracting the cut plant with a solvent selected from the group consisting of water, alcohol, an organic solvent, and mixtures thereof, thereby obtaining an extract; and
(b) filtering the extract and concentrating the filtered extract under reduced pressure, thereby obtaining a *Quamoclit angulata* extract.

8. The method of claim 7, wherein further comprising a step (c) of purifying the *Quamoclit angulata* extract, obtained in step (b), using activated carbon, and concentrating the purified extract under reduced pressure.

9. The method of claim 8, wherein the purifying of step (c) is performed by using an activated carbon-packed chromatography column.

10. A *Quamoclit angulata* extract, which is prepared by a method of any one of claims 7 to 9 and has VEGF inhibitory effects, insulin secretion promoting effects, blood glucose lowering effects, proteinuria (urinary protein) lowering effects, and lenticular opacity reducing effects.

11. A pharmaceutical composition for preventing or delaying aging, which contains a *Quamoclit angulata* extract.

12. The pharmaceutical composition of claim 11, wherein the extract is extracted with a solvent selected from the group consisting of water, alcohol, an organic solvent, and mixtures thereof.

13. A health functional food for preventing or delaying aging, which contains a *Quamoclit angulata* extract.

14. The health functional food of claim 13, wherein the extract is extracted with a solvent selected from the group consisting of water, alcohol, an organic solvent, and mixtures thereof.

15. A pharmaceutical composition for preventing or treating cancer, which contains a *Quamoclit angulata* extract.

16. The pharmaceutical composition of claim 15, wherein the extract is extracted with a solvent selected from the group consisting of water, alcohol, an organic solvent, and mixtures thereof.


<110>    Korea Research Institute of Bioscience and Biotechnology

<120>    Pharmaceutical Compositon for Treating Diabetes Containing
         Extract of Quamoclit angulata

<130>    PP-B0973

<150>    10-2010-0014977
<151>    2010-02-19

<160>    2

<170>    KopatentIn 2.0

<210>    1
<211>    20
<212>    DNA
<213>    Artificial Sequence

<220>
<223>    primer


<400>    1
ttgccttgct gctctacctc                                                   20


<210>    2
<211>    20
<212>    DNA
<213>    Artificial Sequence

<220>
<223>    primer


<400>    2
aaatgctttc tccgctctga                                                   20